# EUROPEAN PATENT SPECIFICATION

(11) **EP 4 105 194 B1**
(45) Date of publication and mention of the grant of the patent: **14.10.2026**
(21) Application number: 21752914.8
(22) Date of filing: 29.01.2021
(51) Int. Cl.: C07C 41/30, C07C 43/23

(54) **METHOD FOR PRODUCING REDUCED COENZYME Q10 FORM II CRYSTALS**
VERFAHREN ZUR HERSTELLUNG VON FORM-II-KRISTALLEN MIT REDUZIERTEM COENZYM Q10
PROCÉDÉ DE PRODUCTION DE CRISTAUX DE COENZYME Q10 RÉDUITE DE FORME II

(30) Priority: 12.02.2020 JP 2020021358
(43) Date of publication of application: 21.12.2022
(73) Proprietor: Kaneka Corporation, Osaka-shi, Osaka 530-8288 (JP)
(72) Inventor: KITAMURA, Shiro, Osaka-shi, Osaka 530-8288 (JP); KINOSHITA, Koichi, Osaka-shi, Osaka 530-8288 (JP); UEDA, Takahiro, Osaka-shi, Osaka 530-8288 (JP)
(74) Representative: Hoffmann Eitle
(86) International application number: PCT/JP2021/003220
(87) International publication number: WO 2021/161807

(56) References cited:
- WO-A1-03/006409
- WO-A1-2009/057611
- WO-A1-2012/176842
- WO-A1-2012/176842
- WO-A1-2020/045571
- WO-A1-2020/045571
- JP-A- 2003 089 669
- HIRAYAMA, NORIAKI: "Passages; Handbook for producing organic compound crystals-Principles and know-how-", 25 July 2008, JP, ISBN: 978-4-621-07991-1, article HIRAYAMA, NORIAKI: "Passages; Handbook for producing organic compound crystals-Principles and know-how-", pages: 17 - 23, 37-40, 45-51, 57- 65, XP009530562
- ETSUO YONEMOCHI: "The topics of pharmaceutical rrvstal oolv form and solubility", JOURNAL OF PHARMACEUTICAL SCIENCE AND TECHNOLOGY, JAPAN, vol. 64, no. 1, 30 November 2003 (2003-11-30), JP, pages 50 - 52, XP009530563, ISSN: 0372-7629, DOI: 10.14843/jpstj.64.50
- HIROSHI OSHIMA: "Chemical Society of Japan", 30 January 2014, JP, ISBN: 978-4-621-08759-6, article MARUZEN CO,: "4.4.3 Crystallization", pages: 269 - 270, XP009530564

## Description

### Technical Field

The present invention relates to a method for producing a reduced coenzyme Q10 Form II-type crystal having excellent stability.

### Background Art

Coenzyme Q is an essential component widely distributed in living organisms from bacteria to mammals, and is known as a component of mitochondrial electron transfer system in cells in the living body. Coenzyme Q serves as a transfer component in the electron transfer system by the repetition of oxidation and reduction in mitochondria, and, further, reduced coenzyme Q is known to have antioxidant activity. The major component in humans is coenzyme Q10 which is one having 10 repeating structures in the side chain of coenzyme Q, and usually, about 40% to 90% thereof is present in the living body as the reduced form. The physiological activity of coenzyme Q includes activation of energy production by mitochondrial activation, activation of cardiac function, an effect of stabilizing cell membranes, and an effect of protecting cells by antioxidant activity.

While coenzyme Q10 currently produced and sold is, in large part, oxidized coenzyme Q10, reduced coenzyme Q10 which exhibits higher oral absorbability than that of oxidized coenzyme Q10 has also been commercially available and has come to be used in recent years.

A common method for obtaining reduced coenzyme Q10 has already been disclosed (Patent Literature 1). Furthermore, several methods for obtaining reduced coenzyme Q10 as a crystal have also been known. For example, a method of crystallizing reduced coenzyme Q10 in an alcohol solution and/or a ketone solution to produce a crystal (Patent Literature 2), a method of adding a high concentration liquid phase of reduced coenzyme Q10 into a poor solvent for crystallization (Patent Literature 3), and the like have been reported.

On the other hand, Patent Literature 4 describes that a crystal polymorphism phenomenon is found in reduced coenzyme Q10. It has been reported that a newly appearing crystal form (wherein this crystal is hereinafter referred to as a "reduced coenzyme Q10 Form II-type crystal" or "Form II-type crystal") is much more stable than the conventional reduced coenzyme Q10 (wherein this crystal is hereinafter referred to as a "reduced coenzyme Q10 Form I-type crystal" or "Form I-type crystal") and also, is excellent in other physical properties.

### Citation List

### Patent Literature

Patent Literature 1: JP Patent Publication No. H10-109933 A (1998)
Patent Literature 2: WO2003/006409
Patent Literature 3: JP Patent Publication No. 2003-089669 A
Patent Literature 4: WO2012/176842

### Summary of Invention

### Technical Problem

Patent Literature 4 describes a method for obtaining a reduced coenzyme Q10 Form II-type crystal, in which crystallization is carried out under specific conditions. However, there is a case where it takes a long period of time and further, the recovered amount is small. Thus, it cannot be necessarily said that this method is industrially optimal. In addition, as a result of the preliminary study, it was found that once Form I-type crystals are precipitated at the time of crystallization, Form I-type crystals are precipitated in preference to Form II-type crystals, and the recovery rate of Form II-type crystals is lowered. Therefore, in order to efficiently obtain reduced coenzyme Q10 Form II-type crystals at high recovery rates, it is necessary to selectively precipitate Form II-type crystals alone.

Accordingly, it is an object of the present invention to provide an efficient production method for obtaining a reduced coenzyme Q10 Form II-type crystal having a stable crystal form.

### Solution to Problem

The present inventors found that the saturated concentration of reduced coenzyme Q10 Form II-type crystals is lower than the saturated concentration of reduced coenzyme Q10 Form I-type crystals in ethanol. Based on this finding, it was found that reduced coenzyme Q10 Form II-type crystals can be obtained efficiently by the following method.
(1) A method for producing a reduced coenzyme Q10 Form II-type crystal, which is characterized by comprising: adding a reduced coenzyme Q10 Form II-type crystal as a seed crystal to a mixed solution containing ethanol and reduced coenzyme Q10; and
   precipitating a reduced coenzyme Q10 Form II-type crystal in the mixed solution after adding the seed crystal,
   wherein a dissolved concentration Cᵢ of reduced coenzyme Q10 in the mixed solution before adding the seed crystal is equal to or more than a saturated concentration of a Form II-type crystal and less than a saturated concentration of a Form I-type crystal at a temperature Tᵢ of the mixed solution when adding the seed crystal, and
   the step of precipitating the crystal comprises controlling a temperature Tₚ of the mixed solution so that a dissolved concentration Cₚ of reduced coenzyme Q10 in the mixed solution is equal to or more than the saturated concentration of the Form II-type crystal and less than the saturated concentration of the Form I-type crystal,
   wherein the ethanol may contain water, an ethanol concentration based on a total volume of water and the ethanol is Z% (v/v), the Z is from 90 to 100, and
   when units of the Cᵢ and the Cₚ are each designated to be % by weight and units of the Tᵢ and the Tₚ are each designated to be K,
   if the Cᵢ and the Cₚ are 1.5% by weight or more,
   all of the following conditions are satisfied: ${A}_{II} ⋅ {T}_{i} + {B}_{II} \leq {logC}_{i} < {A}_{I} ⋅ {T}_{i} + {B}_{I} ,$ (Expression 1) ${A}_{II} ⋅ {T}_{p} + {B}_{II} \leq {logC}_{p} < {A}_{I} ⋅ {T}_{p} + {B}_{I} ,$ (Expression 2) ${A}_{I} = 0.0089 ⋅ Z - 0.6754 ,$ (Expression 3) ${B}_{I} = - 2.3607 ⋅ Z + 172.70 ,$ (Expression 4) ${A}_{II} = 0.0086 ⋅ Z - 0.6844 ,$ (Expression 5) and ${B}_{II} = - 2.3178 ⋅ Z + 178.26 ;$ (Expression 6)
   wherein the Tᵢ is from 20°C to 43°C, and wherein the Tₚ is from 5°C to 43°C.
(2) The production method according to (1), wherein the Tᵢ is from 20°C to lower than 32°C.
(3) The production method according to (1) or (2), wherein precipitation of the crystal comprises maintaining the Tₚ at a constant temperature.
(4) The production method according to any one of (1) to (3), wherein precipitation of the crystal comprises decreasing over time the Tₚ.
(5) The production method according to any one of (1) to (4), wherein precipitation of the crystal comprises:
   maintaining the Tₚ at a constant temperature within a range of 20°C to 43°C and then
   lowering the temperature of the mixed solution to a temperature that is equal to or lower than 25°C and lower than the constant temperature at a rate of -15°C/hour or less.

### Advantageous Effects of Invention

According to the method of the present invention, a reduced coenzyme Q10 Form II-type crystal can be efficiently produced.

### Brief Description of Drawings

[Figure 1] Figure 1 is a graph plotting the solubilities of the Form I-type crystal and the Form II-type crystal in hydrous ethanol at ethanol concentrations of 99.5% (v/v), 95% (v/v), and 90% (v/v) with the temperature (K) on the x-axis and the solubility (% by weight) of the reduced coenzyme Q10 crystal on the y-axis.
[Figure 2] Figure 2 is a graph plotting the solubilities of the Form I-type crystal and the Form II-type crystal in 99.5% (v/v) hydrous ethanol with the temperature (K) on the x-axis and the natural logarithm of the solubility (% by weight) of the reduced coenzyme Q10 crystal on the y-axis.
[Figure 3] Figure 3 is a graph plotting the solubilities of the Form I-type crystal and the Form II-type crystal in 95% (v/v) hydrous ethanol with the temperature (K) on the x-axis and the natural logarithm of the solubility (% by weight) of the reduced coenzyme Q10 crystal on the y-axis.
[Figure 4] Figure 4 is a graph plotting the solubilities of the Form I-type crystal and the Form II-type crystal in 90% (v/v) hydrous ethanol with the temperature (K) on the x-axis and the natural logarithm of the solubility (% by weight) of the reduced coenzyme Q10 crystal on the y-axis.
[Figure 5] Figure 5 is a graph plotting the ethanol concentration on the x-axis and the value of A_{I} on the y-axis to confirm the correlation of the constant A_{I} in the relational expression (Expression 7) logC_{I} = A_{I}•T + B_{I} between temperature T (K) and the solubility (saturated concentration) C_{I} (% by weight) of the Form I-type crystal with the ethanol concentration.
[Figure 6] Figure 6 is a graph plotting the ethanol concentration on the x-axis and the value of B_{I} on the y-axis to confirm the correlation of the constant B_{I} in the above Expression 7 with the ethanol concentration.
[Figure 7] Figure 7 is a graph plotting the ethanol concentration on the x-axis and the value of A_{II} on the y-axis to confirm the correlation of the constant A_{II} in the relational expression (Expression 8) logC_{II} = A_{II}•T + B_{II} between temperature T (K) and the solubility (saturated concentration) C_{I} (% by weight) of the Form I-type crystal with the ethanol concentration.
[Figure 8] Figure 8 is a graph plotting the ethanol concentration on the x-axis and the value of B_{II} on the y-axis to confirm the correlation of the constant B_{II} in the above Expression 8 with the ethanol concentration.

### Description of Embodiments

Hereinafter, the present invention will be described in detail.

### <Reduced coenzyme Q10>

The "reduced coenzyme Q10" herein may partially include oxidized coenzyme Q10, if it includes reduced coenzyme Q10 as a main component. The "main component" herein means that it is included in a proportion of, for example, 50% by weight or more, usually 60% by weight or more, preferably 70% by weight or more, more preferably 80% by weight or more, further preferably 90% by weight or more, particularly preferably 95% by weight or more, and further particularly preferably 98% by weight or more. Herein, the above-described percentage is the percentage of the reduced coenzyme Q10 to the total weight of coenzyme Q10.

Besides, as mentioned above, the reduced coenzyme Q10 includes two types of crystal polymorphisms, namely, the conventionally known Form I-type and a recently newly discovered Form II-type. Specifically, the crystal form of reduced coenzyme Q10 having a melting point around 48°C and showing characteristic peaks at diffraction angles (2θ ± 0.2°) of 3.1°, 18.7°, 19.0°, 20.2° and 23.0° in powder X-ray (Cu-Kα) diffraction is Form I-type, whereas the crystal form of reduced coenzyme Q10 having a melting point around 52°C and showing characteristic peaks at diffraction angles (2θ ± 0.2°) of 11.5°, 18.2°, 19.3°, 22.3°, 23.0° and 33.3° in powder X-ray (Cu-Kα) diffraction is Form II-type. In the present description, the crystal of reduced coenzyme Q10 that satisfies at least one of the following conditions is referred to as a "reduced coenzyme Q10 Form II-type crystal": when the temperature is increased at a rate of 5°C/min by differential scanning calorimetry (DSC), the reduced coenzyme Q10 crystal has an endothermic peak at 54 ± 2°C; the same measurement is carried out at a temperature rising rate of 1°C/min, the reduced coenzyme Q10 crystal has an endothermic peak at 52 ± 2°C; and in powder X-ray (Cu-Kα) diffraction, the reduced coenzyme Q10 crystal shows characteristic peaks at diffraction angles (2θ ± 0.2°) of 11.5°, 18.2°, 19.3°, 22.3°, 23.0° and 33.3°. Of course, it may also be adequate if the reduced coenzyme Q10 crystal satisfies all of these conditions.

Moreover, the "crystalline solid" is used in the present description to mean a solid containing therein an amorphous component having no crystal structure, as well as a portion having a crystal structure.

### <Ethanol>

The present inventors found that the saturated concentration of the Form II-type crystal is smaller than the saturated concentration of the Form I-type crystal in ethanol and further found that the temperature of mixture containing ethanol and reduced coenzyme Q10 is controlled so that the dissolved concentration of reduced coenzyme Q10 is equal to or more than the saturated concentration of the Form II-type crystal and less than the saturated concentration of the Form I-type crystal in ethanol, thereby making it possible to efficiently precipitate the Form II-type crystal.

In the present invention, ethanol may be any solvent containing ethanol as the main component and may be hydrous ethanol containing water. Surprisingly, the difference between the saturated concentration of the Form II-type crystal and the saturated concentration of the Form I-type crystal in ethanol is larger as the water content in ethanol is lower, which makes it easy to selectively precipitate the Form II type crystal under the same temperature conditions. Therefore, it is preferable that the ethanol concentration is, for example, preferably 80% (v/v) or more, 90% (v/v) or more, or 95% (v/v) or more, and 100% (v/v) or less, 99.8% (v/v) or less, or 99.5% (v/v) or less based on the total volume of water and ethanol.

### <Method for Producing Reduced Coenzyme Q10 Form II-type Crystal>

The method for producing a reduced coenzyme Q10 Form II-type crystal according to one or more embodiments of the present invention is characterized by comprising:
adding a reduced coenzyme Q10 Form II-type crystal as a seed crystal to a mixed solution containing ethanol and reduced coenzyme Q10; and
precipitating a reduced coenzyme Q10 Form II-type crystal in the mixed solution after adding the seed crystal,
wherein a dissolved concentration Cᵢ of reduced coenzyme Q10 in the mixed solution before adding the seed crystal is equal to or more than a saturated concentration of a Form II-type crystal and less than a saturated concentration of a Form I-type crystal at a temperature Tᵢ of the mixed solution when adding the seed crystal, and
the step of precipitating the crystal comprises controlling a temperature Tₚ of the mixed solution so that a dissolved concentration Cₚ of reduced coenzyme Q10 in the mixed solution is equal to or more than the saturated concentration of the Form II-type crystal and less than the saturated concentration of the Form I-type crystal.

In the following description, the step of the addition of seed crystals may be referred to as a "seed crystal addition step" and the step of precipitating a reduced coenzyme Q10 Form II-type crystal may be referred to as a "crystal precipitation step."

The mixed solution containing ethanol and reduced coenzyme Q10 is not particularly limited as long as it contains ethanol and reduced coenzyme Q10. It may be a uniform solution in which reduced coenzyme Q10 is dissolved in ethanol or a slurry in which part of reduced coenzyme Q10 is dissolved in ethanol but the other part is not dissolved and is suspended. However, it is preferably a uniform solution in which reduced coenzyme Q10 is dissolved in ethanol.

The reduced coenzyme Q10 used in the mixed solution containing ethanol and reduced coenzyme Q10 may be either a crystalline or amorphous state, and the crystal polymorphism thereof is not limited. Accordingly, the conventionally known reduced coenzyme Q10 Form I-type can also be used. In addition, since the purity of the reduced coenzyme Q10 can be increased in precipitation of a crystal, reduced coenzyme Q10 having impurities, or unpurified or roughly purified reduced coenzyme Q10 may also be used. Furthermore, an extract of the reduced coenzyme Q10 obtained by a conventionally known method, or a reaction solution containing reduced coenzyme Q10 obtained from oxidized coenzyme Q10 by a known reduction method may also be used as the mixed solution, directly, or after purification and/or solvent substitution, as necessary.

The mixed solution containing ethanol and reduced coenzyme Q10 may further comprise a different organic solvent other than the ethanol (including hydrous ethanol). However, the ethanol content per total volume of solvent components is preferably 95% (v/v) or more, 97% (v/v) or more, or 99% (v/v) or more, and the upper limit thereof is preferably 100% (v/v) or less. It is most preferably 100% (v/v). Examples of the other organic solvents include at least one organic solvent selected from the group consisting of an alcohol other than the ethanol, a hydrocarbon, a fatty acid ester, and a nitrogen compound.

The dissolved concentration Cᵢ of the reduced coenzyme Q10 before the addition of seed crystals in the mixed solution containing ethanol and reduced coenzyme Q10 may be equal to or more than the saturated concentration of the Form II-type crystal and less than the saturated concentration of the Form I-type crystal at temperature Tᵢ. The dissolved concentration Cᵢ of the reduced coenzyme Q10 before the addition of seed crystals can be, for example, 2% by weight or more, preferably 3% by weight or more, more preferably 5% by weight or more, even more preferably 7% by weight or more, further preferably 9% by weight or more, still further preferably 10% by weight or more, and can be, for example, 50% by weight or less, preferably 45% by weight or less, more preferably 30% by weight or less.

In the mixed solution containing ethanol and reduced coenzyme Q10, the dissolved concentration Cᵢ of the reduced coenzyme Q10 is equal to or more than the saturated concentration of the Form II-type crystal and less than the saturated concentration of the Form I-type crystal at a temperature Tᵢ of the mixed solution when the addition of seed crystals. Therefore, it is a saturated or supersaturated solution of the Form II-type crystal and an unsaturated solution of the Form I-type crystal. Such a solution is obtained by heating a raw material mixture containing ethanol and reduced coenzyme Q10 to a temperature of 42°C or higher, more preferably 70°C or lower, particularly preferably 55°C or lower so that the reduced coenzyme Q10 is dissolved, and then cooling the heated solution to a temperature Tᵢ to prepare a saturated or supersaturated solution of the Form II-type crystal.

The added amount of reduced coenzyme Q10 Form II-type crystals used as seed crystals (the added amount of seed crystals) is not particularly limited, but it is preferably 0.1% by weight or more, more preferably 0.5% by weight or more, further preferably 0.8% by weight or more, particularly preferably 1% by weight or more based on the amount of reduced coenzyme Q10 in the mixed solution before the addition of seed crystals. The upper limit thereof is not particularly limited, but it is preferably 20% by weight or less, more preferably 4% by weight or less, particularly preferably 2% by weight or less based on the amount of reduced coenzyme Q10 in the mixed solution before the addition of seed crystals. Besides, the reduced coenzyme Q10 crystals used as seed crystals may also comprise reduced coenzyme Q10 Form I-type crystals or amorphous solids, as long as the reduced coenzyme Q10 crystals comprise reduced coenzyme Q10 Form II-type crystals. However, the reduced coenzyme Q10 crystals comprising reduced coenzyme Q10 Form II-type crystals having a high purity are preferable. It is preferable to use, as seed crystals, reduced coenzyme Q10 crystals comprising, for example, 50% by weight or more, preferably 75% by weight or more, even more preferably 80% by weight or more, further preferably 90% by weight or more of reduced coenzyme Q10 Form II-type crystals.

The temperature Tᵢ of the mixed solution when the addition of seed crystals is within a range of 20°C to 43°C. When the temperature Tᵢ is 20°C or higher, the difference between the saturated concentration of the Form II-type crystal and the saturated concentration of the Form I-type crystal is large and the saturated concentration of the Form I-type crystal is high, which is thus appropriate for selective precipitation of the Form II-type crystal. When the temperature Tᵢ is 43°C or lower, the crystal formation rate is high. When the temperature Tᵢ is more preferably 25°C or higher, even more preferably 30°C or higher, further preferably 32°C or higher, still further preferably 35°C or higher, the difference between the saturated concentration of the Form II-type crystal and the saturated concentration of the Form I-type crystal is greater than the above, and the saturated concentration of the Form I-type crystal is high, which thus makes it easier to selectively precipitate the Form II-type crystal. The upper limit of the temperature Tᵢ is more preferably 40°C or lower. When the Tᵢ is from 20°C to lower than 32°C, it is also one preferred embodiment of the present invention.

The crystal precipitation step comprises controlling a temperature Tₚ of the mixed solution after seed crystal addition so that a dissolved concentration Cₚ of reduced coenzyme Q10 in the mixed solution is equal to or more than the saturated concentration of the Form II-type crystal and less than the saturated concentration of the Form I-type crystal.

The temperature Tₚ can be appropriately controlled depending on the dissolved concentration Cₚ of the reduced coenzyme Q10 at that time. The temperature Tₚ is from 5°C to 43°C. In the crystal precipitation step, the temperature Tₚ may be a constant temperature. However, as the dissolved concentration Cₚ of the reduced coenzyme Q10 declines with the precipitation of crystals, it is preferable to carry out cooling crystallization to decrease the temperature Tₚ of the mixed solution over time for the purpose of promoting crystallization. The expression "decrease the temperature Tₚ over time" includes decreasing the temperature Tₚ stepwise or continuously over time.

In the crystal precipitation step, the formation of supersaturation is preferably regulated by controlling the amount of crystals precipitated per unit time. The preferred amount of crystals precipitated per unit time is, for example, the rate of precipitating approximately 50% of the total amount of crystals precipitated per unit time, or lower (i.e., at maximum, 50% amount/hour), and is preferably the rate of precipitating approximately 25% of the total amount of crystals precipitated per unit time, or lower (i.e., at maximum, 25% amount/hour).

One preferred example of the crystal precipitation step includes maintaining the temperature Tₚ at a constant temperature, for example, a temperature range mentioned above as a preferred range of the temperature Tᵢ when the addition of seed crystals, for example, at a constant temperature within a range of 20°C to 43°C. In particular, it is more preferable that the step includes maintaining the temperature Tₚ for 1 hour or longer after the addition of seed crystals within the temperature range mentioned above as a preferred range of the temperature Tᵢ when the addition of seed crystals, for example, at a constant temperature within a range of 20°C to 43°C. The time period of maintaining the mixed solution within the above temperature range is, but is not particularly limited to, preferably 1 hour or longer, more preferably 2 hours or longer, even more preferably 4 hours or longer, particularly preferably 10 hours or longer. The upper limit of the time period of maintaining the mixed solution within the above temperature range is not particularly limited, but sufficient effects can be obtained for approximately 24 hours. Here, the expression "maintaining at a constant temperature" means preferably maintaining at a specific temperature (preset temperature) ± 3°C, more preferably maintaining at a specific temperature (preset temperature) ± 1°C, further preferably maintaining at a specific temperature (preset temperature) ± 0.5°C.

When carrying out cooling crystallization, it is preferable to maintain the temperature Tₚ of the mixed solution within the above temperature after the addition of seed crystals, and then decrease the temperature stepwise or continuously. The rate of decreasing the temperature Tₚ (cooling rate) may be either constant or varied.

The cooling rate when carrying out cooling crystallization is, but is not particularly limited to, for example, a cooling rate at which the temperature drop range per hour is preferably 15°C or lower (= a temperature change rate of -15°C/hour or less), more preferably 10°C or lower, even more preferably 5°C or lower, more preferably 1°C or higher, even more preferably 2°C or higher. One example of carrying out cooling crystallization can be an aspect in which the temperature Tₚ of the mixed solution is maintained within the above temperature after the addition of seed crystals, and then the temperature of the mixed solution is decreased to a temperature that is equal to or lower than 25°C and lower than the above constant temperature at a rate of -15°C/hour or less. When the temperature of the mixed solution is decreased over time, the cooling rate may be either constant or varied. In particular, according to an embodiment in which the above-described cooling rate continuously or stepwise increases, namely, the temperature decrease width per hour increases, as the temperature of the mixed solution decreases, the reduced coenzyme Q10 whose amount remaining in the liquid phase decreases as the temperature of the mixed solution decreases can be efficiently crystallized. For example, until the temperature of the mixed solution reaches 25°C, the mixed solution can be cooled at a rate in which the temperature decrease width per hour is preferably 5°C or lower, and more preferably 3°C or lower. Then, at the stage in which the mixed solution is further cooled to lower than 25°C, the mixed solution can be cooled at a rate in which the temperature decrease width per hour is preferably 6°C or higher, and more preferably 8°C or higher. The end-point temperature when carrying out cooling crystallization is preferably 25°C or lower, more preferably 20°C or lower, even more preferably 10°C or lower, further preferably 7°C or lower, and still further preferably 5°C or lower. The lower limit of the above-described end-point temperature is the solidification temperature of the system of the mixed solution and is preferably 0°C or higher, and more preferably 3°C or higher.

Precipitation of crystals is preferably carried out, while forced-flowing the mixed solution after the addition of seed crystals. In order to suppress the formation of supersaturation and smoothly conduct nucleation and/or crystal growth, or from the viewpoint of the achievement of high quality, flowing may be given to the mixed solution at a power required for stirring per unit volume of generally approximately 0.01 kW/m³ or more, preferably 0.03 kW/m³ or more, more preferably 0.1 kW/m³ or more, and further preferably 0.3 kW/m³ or more. The forced-flowing is generally given by the rotation of a stirring blade. However, if the above-described flowing is obtained, the use of the stirring blade is not always necessary, and for example, a method involving circulation of the mixed solution or the like may be utilized.

the crystallization step may comprise controlling a temperature Tₚ of the mixed solution so that a dissolved concentration Cₚ of reduced coenzyme Q10 is equal to or more than the saturated concentration of the Form II-type crystal and less than the saturated concentration of the Form I-type crystal. For example, during a time period in which preferably 70% by weight or more, more preferably 80% by weight or more, even more preferably 85% by weight or more, further preferably 90% by weight or more of the total amount of the reduced coenzyme Q10 contained in the mixed solution is precipitated as crystals after the addition of seed crystals, or during a time period in which the dissolved concentration Cₚ of reduced coenzyme Q10 reaches 1.5% by weight or less after the addition of seed crystals, if the temperature Tₚ of the mixed solution is controlled to satisfy the above-described conditions, as most of the reduced coenzyme Q10 is precipitated in the form of Form II-type crystals, the temperature conditions may deviate from the above conditions thereafter.

Surprisingly, the present inventors found that when based on the total volume of water and ethanol in the mixed solution, the ethanol concentration is Z% (v/v), the Z is from 90 to 100, the unit of the dissolved concentration C of reduced coenzyme Q10 in the mixed solution is designated to be % by weight, and the unit of temperature T of the mixed solution is designated to be K, the solubility of the Form I-type crystal at a temperature T (K) (saturated concentration) C_{I} (% by weight) and the solubility of the Form II-type crystal at a temperature T (K) (saturated concentration) C_{II} (% by weight) can be expressed by the following Expressions 7 and 8, respectively, where A_{I}, B_{I}, A_{II}, and B_{II} are independently constants. ${logC}_{I} = {A}_{I} ⋅ T + {B}_{I}$ (Expression 7) ${logC}_{II} = {A}_{II} ⋅ T + {B}_{II}$ (Expression 8)

The present inventors further found that each of the constants A_{I}, B_{I}, A_{II}, and B_{II} can be expressed by the following Expressions 3, 4, 5, and 6, respectively, with the ethanol concentration Z as a variable. ${A}_{I} = 0.0089 ⋅ Z - 0.6754$ (Expression 3) ${B}_{I} = - 2.3607 ⋅ Z + 172.70$ (Expression 4) ${A}_{II} = 0.0086 ⋅ Z - 0.6844$ (Expression 5) ${B}_{II} = - 2.3178 ⋅ Z + 178.26$ (Expression 6)

Therefore, in the seed crystal addition step, when the dissolved concentration Cᵢ of the reduced coenzyme Q10 and the temperature Tᵢ of the mixed solution satisfy the following Expression 1, the dissolved concentration Cᵢ of the reduced coenzyme Q10 is equal to or more than the saturated concentration of the Form II-type crystal and less than the saturated concentration of the Form I-type crystal. Here, the unit of Cᵢ is designated to be % by weight, and the unit of Tᵢ is designated to be K. It was found that Expression 1 is established when Cᵢ is equal to or more than 1.5% by weight. ${A}_{II} ⋅ {T}_{i} + {B}_{II} \leq {logC}_{i} < {A}_{I} ⋅ {T}_{i} + {B}_{I}$ (Expression 7)

Similarly, in the crystal precipitation step, when the dissolved concentration Cₚ of the reduced coenzyme Q10 and the temperature Tₚ of the mixed solution satisfy the following Expression 2, the dissolved concentration Cₚ of the reduced coenzyme Q10 is equal to or more than the saturated concentration of the Form II-type crystal and less than the saturated concentration of the Form I-type crystal. Here, the unit of Cₚ is designated to be % by weight and the unit of Tₚ is designated to be K. It was found that Expression 2 is established when Cₚ is equal to or more than 1.5% by weight. According to the present invention as defined in the claims, all of Expressions 1 to 6 are satisfied. ${A}_{II} ⋅ {T}_{p} + {B}_{II} \leq {logC}_{p} < {A}_{I} ⋅ {T}_{p} + {B}_{I}$ (Expression 8)

The reduced coenzyme Q10 Form II-type crystal or crystalline solid obtained by the above method is recovered through a step of solid-liquid separation and drying by a conventionally known method described in, for example, Patent Literature 2 or 3. For example, pressure filtration or centrifugal filtration can be used for solid-liquid separation. In addition, the crystal or crystalline solid after drying can also be recovered by pulverization or classification (sieving), if necessary.

In the present invention, as one of more preferred aspects, after completion of the aforementioned solid-liquid separation, the reduced coenzyme Q10 Form II-type crystal or the crystalline solid is dried with warming, so that the content rate of the reduced coenzyme Q10 Form II-type crystal can be improved. For this purpose, the drying temperature is preferably 46°C or higher, more preferably 47°C or higher, and further preferably 49°C or higher. The upper limit of the drying temperature is generally 52°C or lower, and preferably 51°C or lower. When the drying temperature is lower than 46°C, drying progresses, but the content rate of the reduced coenzyme Q10 Form II-type crystal is hardly improved. On the other hand, when the drying temperature exceeds 52°C, there may be a case where the reduced coenzyme Q10 crystal is melted during the drying. In addition, the warming time in the case of carrying out drying under the aforementioned conditions is not particularly limited, but it is preferably 4 hours or more, more preferably 10 hours or more, and further preferably 20 hours or more.

Besides, when the desired content rate of the reduced coenzyme Q10 Form II-type crystal has already been achieved in the crystal precipitation step, the aforementioned drying conditions shall not apply, and the drying may be carried out at a temperature of, for example, 25°C or higher, preferably 30°C or higher, and more preferably 35°C or higher.

Besides, individual steps in the method of the present invention, specifically, the above-described seed crystal addition step, crystal precipitation step, recovery step such as solid-liquid separation or drying, and other subsequent treatment steps are preferably carried out under a deoxidizing atmosphere. The deoxidizing atmosphere can be achieved by the replacement of the atmosphere with an inert gas, reduction of the pressure, boiling, or a combination thereof. The replacement of the atmosphere with an inert gas, namely, an inert gas atmosphere is preferably used. Examples of the inert gas include nitrogen gas, helium gas, argon gas, hydrogen gas, and carbon dioxide, and preferred is nitrogen gas.

Whether or not the reduced coenzyme Q10 Form II-type crystal is contained in the obtained reduced coenzyme Q10 crystal or crystalline solid, and the content rate thereof can be determined by measuring with, for example, a differential scanning calorimeter (DSC).

As mentioned above, when the reduced coenzyme Q10 Form II-type crystal is measured with DSC at a temperature rising rate of 1°C/min, it exhibits an endothermic peak around 52 ± 2°C. On the other hand, the reduced coenzyme Q10 Form I-type crystal exhibits an endothermic peak around 48 ± 1°C under the same conditions as those described above. Even in a state in which the reduced coenzyme Q10 Form II-type crystal is mixed with the conventional reduced coenzyme Q10 Form I-type crystal or a crystalline solid thereof, the presence or absence of the reduced coenzyme Q10 Form II-type crystal or the content rate thereof can be determined based on the presence or absence of the above-described peak around 52 ± 2°C, the height of the endothermic peak, or the ratio of the endothermic amount. According to the method of the present invention, a reduced coenzyme Q10 Form II-type crystal or a crystalline solid having a high purity can be efficiently obtained.

### Examples

Hereinafter, the present invention will be more specifically described based on Examples. However, the technical scope of the present invention is not intended to be limited to these Examples.

### <Percentage of Reduced Coenzyme Q10 Form II-type Crystal>

Regarding the percentage of Form II-type crystals in recovered reduced coenzyme Q10 crystals, the recovered crystals were analyzed by DSC measurement under the following conditions. From the height (Difference Y) of the endothermic peak of reduced coenzyme Q10 Form I-type crystals (hereinafter referred to as "Difference I-Y") and the height (Difference Y) of the endothermic peak of reduced coenzyme Q10 Form II-type crystals (hereinafter referred to as "Difference II-Y") obtained above, the percentage of the reduced coenzyme Q10 Form II-type crystals (Form II rate) was calculated based on the following formula.

### (DSC Measurement Conditions)

Apparatus: DSC 6220, manufactured by SII Nano Technology Inc.
Sample container: Aluminum pan & cover (SSC000C008)
Rate of temperature rise: 1°C/min
Amount of sample: 5 ± 2 mg
[Formula 1] $Form II rate \left(%\right) = \frac{\left(Difference II-Y\right)}{\left(Difference I-Y\right) + \left(Difference II-Y\right)} \times 100$

### <Measurement of Dissolved Concentration of Reduced Coenzyme Q10 in Liquid Phase>

The dissolved concentration of reduced coenzyme Q10 in a liquid phase was measured by analyze a liquid phase sample by high performance liquid chromatography under the following conditions.

### (HPLC conditions)

Column: SYMMETRY C18 (manufactured by Waters); 250 mm (length), 4.6 mm (inner diameter)
Mobile phase: C₂H₅OH:CH₃OH = 4 : 3 (v : v)
Detection wavelength: 210 nm
Flow rate: 1 ml/min
Retention time of reduced coenzyme Q10: 9.1 min.

### <Experiment 1>

The solubility (saturated concentration) (% by weight) of each of a reduced coenzyme Q10 Form I-type crystal (Form I-type crystal) and a reduced coenzyme Q10 Form II-type crystal (Form II-type crystal) in hydrous ethanol with an ethanol concentration per total volume of 99.5% (v/v), 95% (v/v), and 90% (v/v) at temperatures of 298.15K (25°C), 303.15K (30°C), 308.15K (35°C), and 313.15K (40°C) was obtained.

The solubility was measured by the following procedures.

To approximately 30 g of each hydrous ethanol, approximately 10 g of a Form I-type crystal or a Form II-type crystal was added, the mixture was stirred under a nitrogen atmosphere and maintained for approximately 1 hour at temperatures of 25°C, 30°C, 35°C, and 40°C. Sampling was performed at each temperature and each sample was filtered quickly to obtain a liquid phase sample. Each sample was analyzed under the above HPLC conditions and the solubility was calculated.

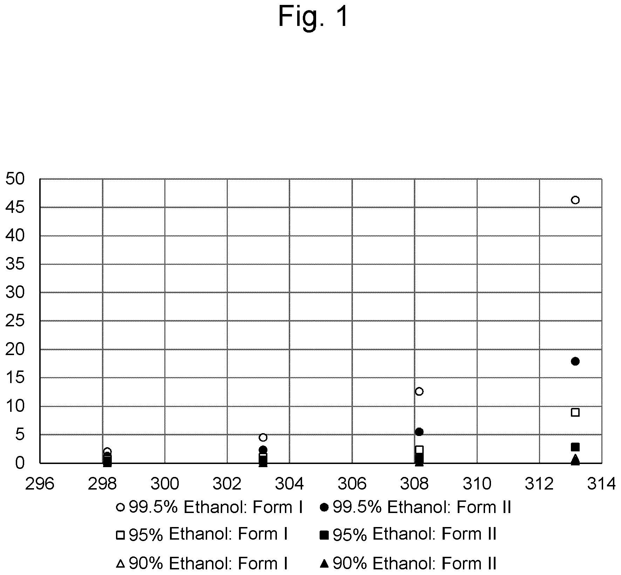

The measurement results are shown in Tables 1 to 3. In addition, Figure 1 shows a graph plotting the results shown in Tables 1 to 3 with the temperature (K) on the x-axis and the solubility (% by weight) of the reduced coenzyme Q10 crystals on the y-axis.

**[Table 1]**

| Solubility in 99.5% (v/v) ethanol | | |
|---|---|---|
| Temperature (K) | Solubility (% by weight) | |
| | Form I | Form II |
| 298.15 | 2.01 | 1.22 |
| 303.15 | 4.53 | 2.32 |
| 308.15 | 12.6 | 5.51 |
| 313.15 | 46.3 | 17.9 |

**[Table 2]**

| Solubility in 95% (v/v) ethanol | | |
|---|---|---|
| Temperature (K) | Solubility (% by weight) | |
| | Form I | Form II |
| 298.15 | 0.65 | 0.37 |
| 303.15 | 1.01 | 0.52 |
| 308.15 | 2.33 | 0.86 |
| 313.15 | 8.98 | 2.81 |

**[Table 3]**

| Solubility in 90% (v/v) ethanol | | |
|---|---|---|
| Temperature (K) | Solubility (% by weight) | |
| | Form I | Form II |
| 298.15 | 0.13 | 0.10 |
| 303.15 | 0.18 | 0.13 |
| 308.15 | 0.29 | 0.23 |
| 313.15 | 0.88 | 0.41 |

As shown in Tables 1 to 3 and Figure 1, it was confirmed that the solubility of the Form I-type crystal is higher than that of the Form II-type crystal in hydrous ethanol with any concentration, and the higher the ethanol concentration, the higher the solubility of each form of crystal.

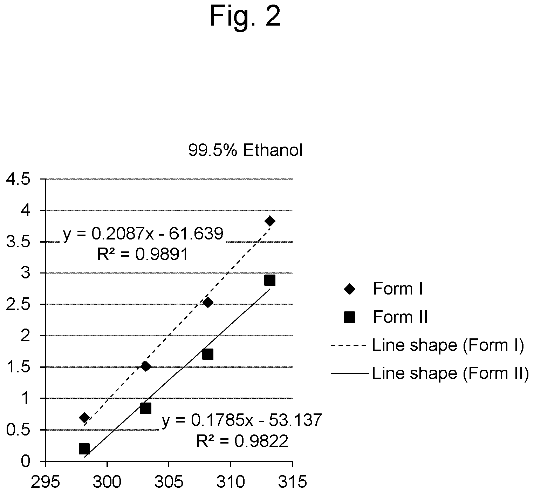

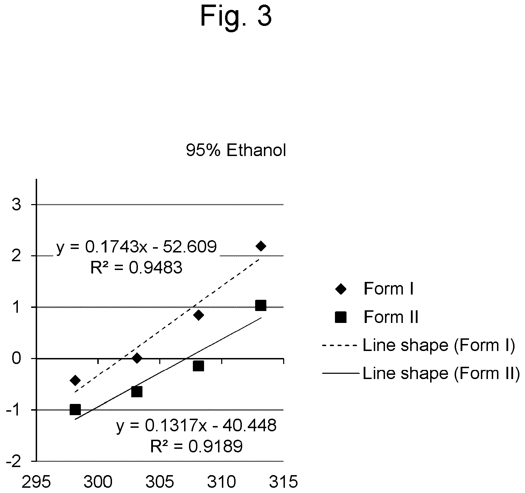

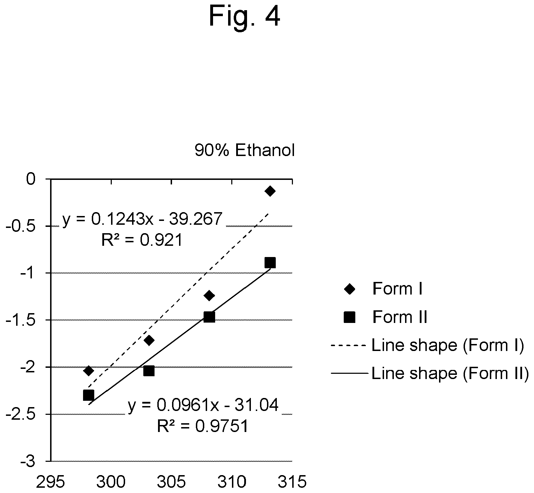

Figures 2, 3, and 4 show graphs plotting the solubilities of the Form I-type crystal and the Form II-type crystal in hydrous ethanol with each concentration shown in Tables 1, 2, and 3, respectively, with the temperature (K) on the x-axis and the natural logarithm of the solubility (% by weight) of the reduced coenzyme Q10 crystal on the y-axis. The expression of the regression line and the correlation coefficient are shown in each graph. Since the correlation coefficient was large enough, the validity of linear regression on a semi-log graph was confirmed. The solubility of the Form I-type crystal at temperature T (K) (saturated concentration) C_{I} (% by weight) and the solubility of the Form II-type crystal at temperature T (K) (saturated concentration) C_{II} (% by weight) in hydrous ethanol with each concentration can be expressed by the following Expressions 7 and 8, respectively, where A_{I}, B_{I}, A_{II}, and B_{II} are independently constants. ${logC}_{I} = {A}_{I} ⋅ T + {B}_{I}$ (Expression 7) ${logC}_{II} = {A}_{II} ⋅ T + {B}_{II}$ (Expression 8)

The values of A_{I}, B_{I}, A_{II}, and B_{II} at the respective ethanol concentrations are shown below.

**[Table 4]**

| Ethanol concentration % (v/v) | A_{I} | B_{I} | A_{II} | B_{II} |
|---|---|---|---|---|
| 99.5 | 0.2087 | -61.639 | 0.1785 | -53.137 |
| 95 | 0.1743 | -52.609 | 0.1317 | -40.448 |
| 90 | 0.1243 | -39.267 | 0.0961 | -31.040 |

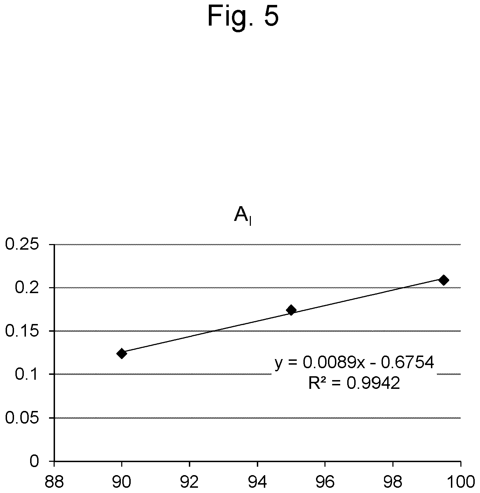

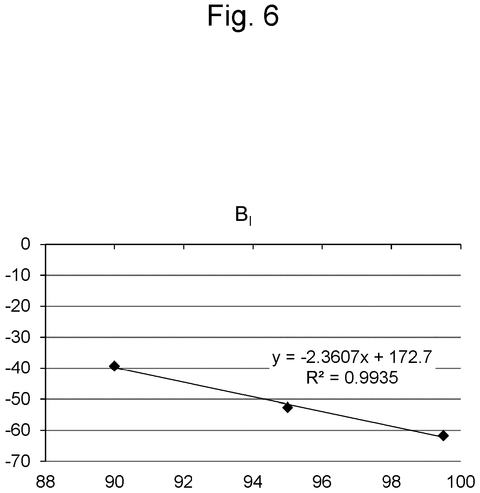

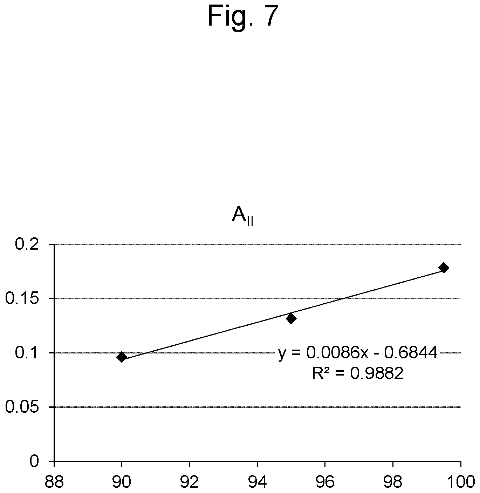

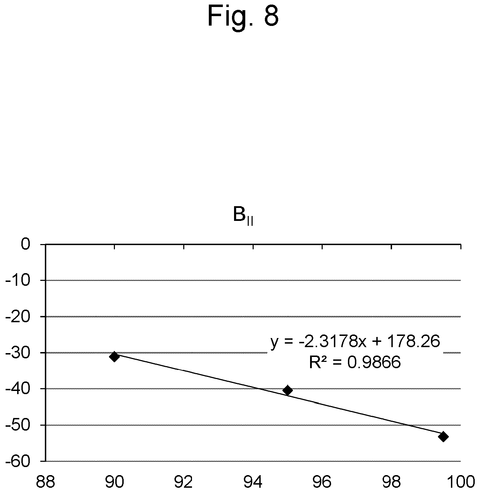

Figures 5, 6, 7, and 8 show graphs plotting the ethanol concentration on the x-axis and the value of each constant on the y-axis to confirm the correlation of A_{I}, B_{I}, A_{II}, and B_{II} with the ethanol concentration. In addition, the regression line and the correlation coefficient are shown in each figure.

It was confirmed that A_{I} and A_{II} are directly proportional to the changes in the concentration of hydrous ethanol, and B_{I} and B_{II} are inversely proportional to changes in the concentration of hydrous ethanol. These results confirmed that the values of the constants A_{I}, B_{I}, A_{II}, and B_{II}, and the ethanol concentration Z (% (v/v)) have a relationship represented by Expressions 3, 4, 5, and 6, respectively.

### <Experiment 2>

The inside of a separable flask (made of borosilicate glass) with a volume of 500 mL was replaced with nitrogen, and 40.0 g of reduced coenzyme Q10 and 360 g of 99.5% ethanol were then charged thereto (reduced coenzyme Q10 concentration: 10 wt%). Thereafter, the obtained mixture was warmed to 42°C while stirring with a stirring blade (power required for stirring: 0.1 kw/m³) to obtain a homogeneous solution. This solution was cooled to 35°C, and thereafter, 0.4 g (1 wt%) of reduced coenzyme Q10 crystals comprising reduced coenzyme Q10 Form II-type crystals were added as seed crystals to the reaction solution.

The mixed solution obtained after the addition of seed crystals was kept at 35°C for 15 hours to precipitate crystals. Thereafter, the mixed solution was cooled to 25°C at a constant cooling rate of -2°C/hour over 5 hours, and was then cooled to 10°C at a constant cooling rate of -10°C/hour over 1.5 hours, to further precipitate crystals. After the temperature had reached 10°C, the mixed solution was filtrated for solid-liquid separation. The obtained crystal was dried under reduced pressure at 35°C for 10 hours to obtain a reduced coenzyme Q10 Form II-type crystal (Form II rate: 100%; recovery rate: 97%).

In the present experiment, the liquid phase of the mixed solution was sampled immediately before the addition of seed crystals (at 0 hour), and then at 3 hours, 6 hours, 9 hours, 12 hours, 15 hours (so far, at 35°C), 17.5 hours (30°C), 20 hours (25°C), 20.5 hours (20°C), and 21.5 hours (10°C) after addition of the seed crystals. The dissolved concentration C (wt%) of reduced coenzyme Q10 in the liquid phase sample was measured according to high performance liquid chromatography. The measurement results are shown in Table 5. Table 5 also shows the natural logarithm LogC of the measured dissolved concentration C.

Further, the ethanol concentration of 99.5% (v/v) was assigned to Z of Expressions 3, 4, 5, and 6 to obtain the values of the constants A_{I}, B_{I}, A_{II}, and B_{II}. Using these constants, the LogC_{I} of the saturated concentration C_{I} (% by weight) of the Form I-type crystal and the LogC_{II} of the saturated concentration C_{II} (% by weight) of the Form II-type crystal were obtained at each temperature (converting the unit to Kelvin) shown in Table 5 according to Expressions 7 and 8 above.

**[Table 5]**

| Crystallization time (h) | Temperature (°C) | Dissolved concentration C of reduced coenzyme Q10 (% by weight) | LogC | LogC_{I} | LogC_{II} |
|---|---|---|---|---|---|
| 0 | 35 | 10.0 | 2.303 | 2.568 | 0.425 |
| 3 | 35 | 9.9 | 2.293 | 2.568 | 0.425 |
| 6 | 35 | 9.6 | 2.262 | 2.568 | 0.425 |
| 9 | 35 | 9.2 | 2.219 | 2.568 | 0.425 |
| 12 | 35 | 8.5 | 2.140 | 2.568 | 0.425 |
| 15 | 35 | 7.2 | 1.974 | 2.568 | 0.425 |
| 17.5 | 30 | 3.1 | 1.131 | 1.517 | -0.432 |
| 20 | 25 | 1.5 | 0.405 | 0.467 | -1.288 |
| 20.5 | 20 | 0.8 | -0.223 | -0.584 | -2.145 |
| 21.5 | 10 | 0.3 | -1.204 | -2.686 | -3.858 |

At the time of the addition of seed crystals at 35°C and a time point up to 20 hours of crystallization after maintaining the temperature at 35°C and then cooling to 25°C, the dissolved concentration C of reduced coenzyme Q10 in the liquid phase was equal to or more than the saturated concentration C_{II} of the Form II-type crystal and less than the saturated concentration C_{I} of the Form I-type crystal. At time points of 20.5 hours (20°C) and 21.5 hours (10°C) of crystallization, the dissolved concentration C exceeded the saturated concentration Ci of the Form I-type crystal while at a time point of 20 hours of crystallization, the dissolved concentration C of reduced coenzyme Q10 in the liquid phase decreased to as low as 1.5% by weight. Since crystal precipitation of most of the reduced coenzyme Q10 dissolved at the time of the addition of seed crystals took place under conditions that allowed the dissolved concentration C in the liquid phase to be equal to or more than the saturated concentration C_{II} of the Form II-type crystal and less than the saturated concentration C_{I} of the Form I-type crystal, Form II-type crystals were obtained at a rate of 100%.

## Claims

1. A method for producing a reduced coenzyme Q10 Form II-type crystal, which is **characterized by** comprising: adding a reduced coenzyme Q10 Form II-type crystal as a seed crystal to a mixed solution containing ethanol and reduced coenzyme Q10; and
precipitating a reduced coenzyme Q10 Form II-type crystal in the mixed solution after adding the seed crystal,
wherein a dissolved concentration Cᵢ of reduced coenzyme Q10 in the mixed solution before adding the seed crystal is equal to or more than a saturated concentration of a Form II-type crystal and less than a saturated concentration of a Form I-type crystal at a temperature Tᵢ of the mixed solution when adding the seed crystal, the Tᵢ is from 20°C to 43°C, and
the step of precipitating the crystal comprises controlling a temperature Tₚ of the mixed solution so that a dissolved concentration Cₚ of reduced coenzyme Q10 in the mixed solution is equal to or more than the saturated concentration of the Form II-type crystal and less than the saturated concentration of the Form I-type crystal, the Tₚ is from 5°C to 43°C,
the Form I-type is the crystal form of reduced coenzyme Q10 having a melting point around 48°C and showing characteristic peaks at diffraction angles (2θ ± 0.2°) of 3.1°, 18.7°, 19.0°, 20.2° and 23.0° in powder X-ray (Cu-Kα) diffraction,
the Form II-type is the crystal form of reduced coenzyme Q10 having a melting point around 52°C and showing characteristic peaks at diffraction angles (2θ ± 0.2°) of 11.5°, 18.2°, 19.3°, 22.3°, 23.0° and 33.3° in powder X-ray (Cu-Kα) diffraction,
the ethanol may contain water, an ethanol concentration based on a total volume of water and the ethanol is Z% (v/v), the Z is from 90 to 100, and
when units of the Cᵢ and the Cₚ are each designated to be % by weight and units of the Tᵢ and the Tₚ are each designated to be K,
if the Cᵢ and the Cₚ are 1.5% by weight or more,
all of the following conditions are satisfied: ${A}_{II} ⋅ {T}_{i} + {B}_{II} \leq {logC}_{i} < {A}_{I} ⋅ {T}_{i} + {B}_{I} ,$ (Expression 1) ${A}_{II} ⋅ {T}_{p} + {B}_{II} \leq {logC}_{p} < {A}_{I} ⋅ {T}_{p} + {B}_{I} ,$ (Expression 2) ${A}_{I} = 0.0089 ⋅ Z - 0.6754 ,$ (Expression 3) ${B}_{I} = - 2.3607 ⋅ Z + 172.70 ,$ (Expression 4) ${A}_{II} = 0.0086 ⋅ Z - 0.6844 , and$ (Expression 5) ${B}_{II} = - 2.3178 ⋅ Z + 178.26 .$ (Expression 6)

2. The production method according to claim 1, wherein the Tᵢ is from 20°C to lower than 32°C.

3. The production method according to claim 1 or 2, wherein precipitation of the crystal comprises maintaining the Tₚ at a constant temperature.

4. The production method according to any one of claims 1 to 3, wherein precipitation of the crystal comprises decreasing over time the Tₚ.

5. The production method according to any one of claims 1 to 4, wherein precipitation of the crystal comprises:
maintaining the Tₚ at a constant temperature within a range of 20°C to 43°C and then
lowering the temperature of the mixed solution to a temperature that is equal to or lower than 25°C and lower than the constant temperature at a rate of -15°C/hour or less.

## Patentansprüche

1. Verfahren zur Herstellung eines Form-II-Kristalls mit reduziertem Coenzym Q10, **dadurch gekennzeichnet, dass** es umfasst: Zusetzen eines Form-II-Kristalls mit reduziertem Coenzym Q10 als Impfkristall zu einer Mischlösung, die Ethanol und reduziertes Coenzym Q10 enthält; und
Ausfällen eines Form-II-Kristalls mit reduziertem Coenzym Q10 in der Mischlösung nach dem Zusetzen des Impfkristalls,
wobei eine gelöste Konzentration Cᵢ von reduziertem Coenzym Q10 in der Mischlösung vor dem Zusetzen des Impfkristalls gleich oder höher als eine Sättigungskonzentration eines Form-II-Kristalls und niedriger als eine Sättigungskonzentration eines Form-I-Kristalls bei einer Temperatur Tᵢ der Mischlösung beim Zusetzen des Impfkristalls ist, wobei das Tᵢ von 20°C bis 43°C reicht, und
der Schritt des Ausfällens des Kristalls das Steuern einer Temperatur Tₚ der Mischlösung umfasst, sodass eine gelöste Konzentration Cₚ von reduziertem Coenzym Q10 in der Mischlösung gleich oder höher als die Sättigungskonzentration des Form-II-Kristalls und niedriger als die Sättigungskonzentration des Form-I-Kristalls ist, wobei das Tₚ von 5°C bis 43°C reicht,
der Form-I-Kristall die Kristallform von reduziertem Coenzym Q10 ist, die einen Schmelzpunkt von etwa 48°C aufweist und charakteristische Spitzen bei Beugungswinkeln (2θ ± 0,2°) von 3,1°, 18,7°, 19,0°, 20,2° und 23,0° in der Pulver-Röntgendiffraktion (Cu-Kα) zeigt,
der Form-II-Kristall die Kristallform von reduziertem Coenzym Q10 ist, die einen Schmelzpunkt von etwa 52°C aufweist und charakteristische Spitzen bei Beugungswinkeln (2θ ± 0,2°) von 11,5°, 18,2°, 19,3°, 22,3°, 23,0° und 33,3° in der Pulver-Röntgendiffraktion (Cu-Kα) zeigt,
das Ethanol Wasser enthalten kann, wobei eine Ethanolkonzentration bezogen auf das Gesamtvolumen von Wasser und Ethanol Z Vol.-% ist, Z im Bereich von 90 bis 100 liegt, und
wenn Einheiten des Cᵢ und des Cₚ jeweils als Gew.-% bezeichnet sind und Einheiten des Tᵢ und des Tₚ jeweils als K bezeichnet sind,
wenn Cᵢ und Cₚ 1,5 Gew.-% oder mehr sind,
alle folgenden Bedingungen erfüllt sind: ${A}_{II} ⋅ {T}_{i} + {B}_{II} \leq {logC}_{i} < {A}_{I} ⋅ {T}_{i} + {B}_{I} ,$ (Gleichung 1) ${A}_{II} ⋅ {T}_{p} + {B}_{II} \leq {logC}_{p} < {A}_{I} ⋅ {T}_{p} + {B}_{I} ,$ (Gleichung 2) ${A}_{I} = 0 , 0089 ⋅ Z - 0 , 6754 ,$ (Gleichung 3) ${B}_{I} = - 2 , 3607 ⋅ Z + 172 , 70 ,$ (Gleichung 4) ${A}_{II} = 0 , 0086 ⋅ Z + 0 , 6844 , und$ (Gleichung 5) ${B}_{II} = - 2 , 3178 ⋅ Z + 178 , 26 .$ (Gleichung 6)

2. Herstellungsverfahren nach Anspruch 1, wobei Tᵢ im Bereich von 20°C bis niedriger als 32°C liegt.

3. Herstellungsverfahren nach Anspruch 1 oder 2, wobei die Ausfällung des Kristalls das Halten von Tₚ auf einer konstanten Temperatur umfasst.

4. Herstellungsverfahren nach einem der Ansprüche 1 bis 3, wobei die Ausfällung des Kristalls das Verringern von Tₚ über die Zeit umfasst.

5. Herstellungsverfahren nach einem der Ansprüche 1 bis 4, wobei die Ausfällung des Kristalls umfasst:
Halten von Tₚ auf einer konstanten Temperatur innerhalb eines Bereichs von 20°C bis 43°C und dann
Absenken der Temperatur der Mischlösung auf eine Temperatur, die gleich oder niedriger als 25°C und niedriger als die konstante Temperatur ist, mit einer Rate von -15°C/Stunde oder weniger.

## Revendications

1. Procédé de production d'un cristal de coenzyme Q10 réduite de type forme II, **caractérisé en ce qu'**il comprend : ajouter un cristal de coenzyme Q10 réduite de type forme II en tant que germe cristallin à une solution mixte contenant de l'éthanol et de la coenzyme Q10 réduite ; et
faire précipiter un cristal de coenzyme Q10 réduite de type forme II dans la solution mixte après avoir ajouté le germe cristallin,
dans lequel une concentration dissoute Cᵢ de coenzyme Q10 réduite dans la solution mixte avant l'ajout du germe cristallin est égale à ou supérieure à une concentration saturée d'un cristal de type forme II et inférieure à une concentration saturée d'un cristal de type forme I à une température Tᵢ de la solution mixte au moment de l'ajout du germe cristallin, le Tᵢ va de 20 °C à 43 °C, et
l'étape consistant à faire précipiter le cristal comprend un contrôle d'une température Tₚ de la solution mixte de sorte qu'une concentration dissoute Cₚ de coenzyme Q10 réduite dans la solution mixte soit égale à ou supérieure à la concentration saturée du cristal de type forme II et inférieure à la concentration saturée du cristal de type forme I, le Tₚ va de 5 °C à 43 °C,
le type forme I est la forme cristalline de la coenzyme Q10 réduite ayant un point de fusion autour de 48 °C et présentant des pics caractéristiques aux angles de diffraction (2θ ± 0,2°) de 3,1°, 18,7°, 19,0°, 20,2° et 23,0° dans une diffraction aux rayons X sur poudre (Cu-Kα),
le type forme II est la forme cristalline de la coenzyme Q10 réduite ayant un point de fusion autour de 52 °C et présentant des pics caractéristiques aux angles de diffraction (2θ ± 0,2°) de 11,5°, 18,2°, 19,3°, 22,3°, 23,0° et 33,3° dans une diffraction aux rayons X sur poudre (Cu-Kα),
l'éthanol peut contenir de l'eau, une concentration en éthanol sur la base d'un volume total d'eau et de l'éthanol est Z % (v/v), le Z va de 90 à 100, et
lorsque les unités du Cᵢ et du Cₚ sont chacune désignées comme étant un % en poids et que les unités du Tᵢ et du Tₚ sont chacune désignées comme étant K,
si le Cᵢ et le Cₚ sont de 1,5 % en poids ou plus,
toutes les conditions suivantes sont satisfaites : ${A}_{II} ⋅ {T}_{i} + {B}_{II} \leq {logC}_{i} < {A}_{I} ⋅ {T}_{i} + {B}_{l} ,$ (Expression 1) ${A}_{II} ⋅ {T}_{p} + {B}_{II} \leq {logC}_{p} < {A}_{I} ⋅ {T}_{p} + {B}_{I} ,$ (Expression 2) ${A}_{I} = 0 , 0089 ⋅ Z - 0 , 6754 ,$ (Expression 3) ${B}_{I} = - 2 , 3607 ⋅ Z + 172 , 70 ,$ (Expression 4) ${A}_{II} = 0 , 0086 ⋅ Z - 0 , 6844 , et$ (Expression 5) ${B}_{II} = - 2 , 3178 ⋅ Z + 178 , 26 .$ (Expression 6)

2. Procédé de production selon la revendication 1, dans lequel le Tᵢ va de 20 °C à moins de 32 °C.

3. Procédé de production selon la revendication 1 ou la revendication 2, dans lequel la précipitation du cristal comprend le fait de maintenir le Tₚ à une température constante.

4. Procédé de production selon l'une quelconque des revendications 1 à 3, dans lequel la précipitation du cristal comprend une diminution au fil du temps du Tₚ.

5. Procédé de production selon l'une quelconque des revendications 1 à 4, dans lequel la précipitation du cristal comprend :
le fait de maintenir le Tₚ à une température constante dans une plage de 20 °C à 43 °C et puis
le fait d'abaisser la température de la solution mixte jusqu'à une température qui est égale à ou inférieure à 25 °C et inférieure à la température constante à une vitesse de -15 °C/heure ou moins.
